# EUROPEAN PATENT APPLICATION

(11) **EP 1 443 084 A1**
(43) Date of publication of application: **04.08.2004**
(21) Application number: 04000817.9
(22) Date of filing: 16.01.2004
(51) Int. Cl.: C09C 1/00, C09D 5/36, C09D 11/02, C09D 11/00, C09K 11/08

(54) **Pearlescent pigments based on fluorides, oxyfluorides, fluorosulfides and/or oxyfluorosulfides**

(30) Priority: 03.02.2003 EP 03002303
(71) Applicant: MERCK PATENT GmbH, 64293 Darmstadt (DE)
(72) Inventor: Reynders, Peter, Dr., 64347 Griesheim (DE); Bertaux, Stephane, Dr., 91100 Villabé (FR); Wichmann, Jens-Uwe, Dr., 79117 Freiburg (DE)

(57) **Abstract**

The present invention relates to novel pigments based on substrates coated with at least one selectively light absorbing layer of fluorides, oxyfluorides, fluorosulfides and/or oxyfluorosulfides. Metal oxides or mixed metal oxides are coated onto a substrate; the resulting precursor is transferred into a furnace and calcined under a fluoridizing and/or sulfurizing gas flow to convert the oxides into fluorides, oxyfluorides, fluorosulfides and/or oxyfluorosulfides depending on the reaction parameters. The conversion to fluorides, oxyfluorides, fluorosulfides and/or oxyfluorosulfides is preferably carried out in a fluidized bed reactor. Angle-dependent optical pigments are thus produced which are especially useful in paints, powder coatings, paper coatings, plastics, cosmetics, inks and security-enhancing features as well as in decorative applications for foods and drugs.

## Description

The present invention relates to novel pigments based on substrates coated with at least one selectively light absorbing layer of fluorides, oxyfluorides, fluorosulfides and/or oxyfluorosulfides. Metal oxides, hydroxides, mixed metal oxides and/or mixed metal hydroxides are coated onto a substrate; the resulting precursor is transferred into a furnace and calcined under a fluoridizing and/or sulfurizing gas flow to convert the oxides into fluorides, oxyfluorides, fluorosulfides and/or oxyfluorosulfides depending on the reaction parameters. The conversion to fluorides, oxyfluorides, fluorosulfides and/or oxyfluorosulfides is preferably carried out in a fluidized bed reactor. Angle-dependent optical pigments are thus produced which are especially useful in paints, powder coatings, paper coatings, plastics, cosmetics, inks and security-enhancing features as well as in decorative applications for foods and drugs.

Absorption pigments based on fluorides and oxyfluorides are well known for many years. A good overview about these substances can be found in Gerken, M. et al, Syntheses and structures of the oxide fluorides of the main-group and transition metal elements, Chapter 5, Advanced Inorganic fluorides: Synthesis, characterization and applications, T.Nakajima, B.Zemva and A. Tressaud (Editors), 2000, Elsevier Science S.A.

US-A 4,256,508 describes improved transparent iron oxide pigments having increased color strength. Such pigments are achieved by the incorporation of fluoride with the crystals or particles of the iron oxide. These pigments may be prepared by the process comprising reacting an aqueous solution of a water-soluble iron salt with an alkaline precipitating agent wherein a fluoride salt is added to the solution or to the reaction product. The fluoride salt is selected from the group consisting of sodium fluoride, and potassium fluoride. By this method only yellow iron oxide pigments are obtainable. The pigments described in this prior art reference do not show desirable multiple color effects.

It was therefore an object of the present invention to provide pigments with a great variety of different mass-tones which combine an viewing angle dependant interference phenomenon with the absorption color, therewith extending the range of pearlescent pigments based on fluorides, oxyfluorides, fluorosulfides and/or oxyfluorosulfides.

The pigments according to the present invention can surprisingly fulfill the above-mentioned object. Therefore, the present invention describes pearlescent pigments based on substrates comprising at least one selectively light absorbing layer, which consists of a fluoride, oxyfluoride, fluorosulfide and/or oxyfluorosulfide.

The synthesis of the new pigments is performed via a two-step process. The first step is the synthesis of a precursor based on a substrate. The second step is a conversion process of the precursor, carried out in a furnace. The pearlescent pigments according to the present invention can be produced in conventional static ovens, belt kilns or rotary kilns. However, a commercially more attractive product with less agglomerates and faster reaction rates is obtained in fluidized bed reactors.

In the first step a layer of an oxide, hydroxide, mixed oxide and/or mixed hydroxide is deposited onto a substrate, thus obtaining the precursor being used in the second step. All known deposition techniques, such as aqueous precipitation processes, CVD and/or PVD processes can be used. However, preferably an aqueous precipitation process described for example in US-A 3,087,828, US-A 3,087,829, DE-A 19 59 998, DE-A 20 09 566, DE-A 22 14 545, DE-A 22 44 298, DE-A 23 13 331, DE-A 25 22 572, DE-A 31 37 808, DE-A 31 37 809, DE-A 31 51 343, DE-A 31 51 354, DE-A 31 51 355, DE-A 32 11 602, DE-A 32 35 107, WO 93/08237 and EP-A 0 763 573 is used to obtain the precursor. Halide, carbonate, oxalate, chloride or oxychloride solutions are used to precipitate oxides, hydroxides, mixed oxides and/or mixed hydroxides onto the substrates. The reaction parameters such as temperature, pH, and agitation velocity and reactor geometry are optimized to yield a flat continuous layer of the insoluble oxides and/or hydroxides on the substrates. The mixed oxides and/or hydroxides are co-precipitated onto the substrates following an analogous process. Solutions of the different metal salts are mixed and then slowly added in the reactor to coat the substrate. The oxide, hydroxide, mixed oxide and/or mixed hydroxide can be doped with metal ions, silicon oxide, aluminum oxide, sulfur, phosphate ions and/or sulfate ions. The dopants can be used to create color effects (like rare earths, vanadium, or cobalt ions) as well as for the control of grain growth (like SiO₂ or aluminum oxide) during the subsequent second step. Examples of metal ions as dopant are silicon, vanadium, chromium, aluminum, cerium, neodymium, praseodymium, selenium, cobalt, nickel and/or zinc ions, preferably vanadium and/or cobalt ions.

Substrates that can be used in the present invention as base material on which the oxides, hydroxides, mixed oxides and/or mixed hydroxides are precipitated include platelet-shaped, spherical or needle-shaped substrates. Preferably the substrates comprise but are not limited to:
Platelets: Micaceous iron oxide, natural (for example as in WO 99/48634), synthetic or doped (for example as in EP-A 0 068 311) micas (muscovite, phlogopite, fluorophlogopite, synthetic fluorophlogopite, talc, kaolin), SiO₂- Al₂O₃-, TiO₂- Glass-, ZnO-, ZrO₂- SnO₂-flakes, pearlescent pigments (including those which react under the fluidized bed conditions to nitrides, oxynitrides or by reduction to suboxides etc.) (for example EP-A 9 739 066, EP-A 0 948 571, WO 99/61529, EP-A 1 028 146, EP-A 0 763 573, US-A 5,858,078, WO 98/53012, WO 97/43348, US-A 6,165,260, DE-A 15 19 116, WO 97/46624, EP-A 0 509 352), pearlescent multilayer pigments (for example EP-A 0 948 572, EP-A 0 882 099, US-A 5,958,125, US-A 6,139,613).
Spheres: Coated SiO₂ spheres (for example EP-A 0 803 550, EP-A 1 063 265, JP-A 11 322 324), uncoated SiO₂ spheres (Ronaspheres®, all spheres described as starting materials in EP-A 0 803 550, EP-A 1 063 265, JP-A 11 322 324), micro bubbles (US-A 4,985,380), as well as needle-shaped metal oxides, preferably iron oxide.

The size of the substrates is not critical. The mean diameter of the substrates and hence the resulting pigments can vary between 1 and 500 µm, preferably 5 and 50 µm. Preferably, the mean diameter in the case of for example the platelet-shaped substrates can vary between 5 and 200 µm, preferably between 10 and 150 µm. The mean diameter of the spherical substrates can vary between 10 nm and 100 µm, preferably between 500 nm and 50 µm and most preferably from 1 to 20 µm. Such substrates are commercially available or can be obtained by known processes.

The substrates to be coated with selectively light absorbing fluorides, oxyfluorides, fluorosulfides and/or oxyfluorosulfides layers can consist themselves of layered structures such as gold-type multilayer pigments (Iriodin Star Gold® , Merck KGaA). The outer layer then can be a low refractive coating with a refractive index of ≤ 1.8, preferably ≤ 1.6. Particularly suitable examples for such a layer material is magnesium fluoride, aluminum phosphate, silicon oxide, silicon oxide hydrate, aluminum oxide, aluminum oxide hydrate and mixtures thereof. The thickness of this layer is generally from 20 to 800 nm, preferably from 50 to 600 nm.

The fluorides, oxyfluorides, fluorosulfides and/or oxyfluorosulfides layer can be coated directly onto the substrate, as described above. In addition the substrates can be pre-coated by a colorless transparent or semitransparent optical layer with a refractive index > 1.8, such as titanium dioxide, titanium suboxides and/or iron oxide. Accordingly, the substrates can be pre-coated by an optical layer of mixed iron oxide-titanium oxide phases, especially pseudobrookite or ilmenite. The precipitation process to coat the substrate is the same as described above.

In the second step for the production of pigments according to the present invention, the oxides, hydroxides, mixed oxides and/or mixed hydroxides obtained in the above-described first step are converted into fluorides, oxyfluorides, fluorosulfides and/or oxyfluorosulfides. This can be achieved by calcination of the precursor obtained in the first step in conventional static ovens, belt kilns or rotary kilns. However, a better product with less agglomerates and faster reaction rates is obtained in fluidized bed reactors. This process can be performed batchwise or continuously. The conversion is carried out with a reactive gas which may consist of fluorine and/or HF, alone or in combination with H₂S, CS₂, sulfur or a mixture thereof. Preferably HF alone or in combination with H₂S is used, in the case of HF in combination with H₂S most preferably in sequenced steps. Additionally an inert gas such as Ar or N₂, preferably N₂ may be present during the conversion. The gas composition may vary from >0 to 100 vol-%, preferably from 20 to 80 vol-% of reaction gas in inert gas.

The temperature is maintained at a fluidized bed temperature of about 700-1250°C, preferably 800°C to 1100°C. The conversion between oxides, hydroxides, mixed oxides as well as mixed hydroxides and fluorides, oxyfluorides, fluorosulfides and/or oxyfluorosulfides is carried out depending on different parameters, such as gas flow rates, reaction time or temperature profiles. The longer the reaction time the higher the fluoride-to-oxyfluoride ratio. Consequently the reaction time determines the obtained structure of the compound. The color and the color strength of the pearlesent pigments according to the present invention is associated to a determined structure that is why the reaction time has to be well controlled. In addition for the same reason the temperature control is necessary. The control and optimization of the process parameters can be performed by any person skilled in the art.

In order to maintain the almost ideal conditions prevalent in an homogeneous fluid bed in comitercurrent/cocurrent, contacting special devices are used. Instabilities like formation of channels or of bubbles in the bed are instantly destroyed by vibrations or agitating facilities.

If the reaction with the reaction gas is not carried out to full completeness, mixtures of phases can be obtained including gradient of phase concentration through the layer thickness. These incompletely reacted products can be advantageous with respect to a desired color shade.

The thickness of the fluorides, oxyfluorides, fluorosulfides and/or oxyfluorosulfides layers can vary between 5 and 500 nm, yielding slight shades and flat angle color effect at low thickness and very pronounced hiding at high thickness. For the optimal interference effect, the preferred thicknesses are 50-350 nm, especially preferred 80-200 nm.

The interference color is determined by the optical thickness, which is the geometrical thickness of the layer multiplied by the refractive index (Pfaff, G., Reynders, P. "Angle-dependent optical effects deriving from submicron structures of films and pigments", Chemical Review, 99 (1999), p.1963-1981). The latter is a strong function of the chosen material but is in general not known for the rather new materials mentioned in this invention. The mass tone of the absorbing pigments is as well a function of the layer thickness. Therefore, the desired color effect is empirically optimized by adjustment of the amount of precursor, by this means controlling the precursor layer thickness, and regulation of the conversion reaction with the reactive gases.

Preferably, the selectively light absorbing layer of fluorides, oxyfluorides, fluorosulfides and/or oxyfluorosulfides may consist of:

### Fluorides:

### 1. Binary fluorides

- AₓF_{y} or hydroxyfluorides, with x>0, y>0 and with A = Fe, Mg, Ca, Ta, Cd, Sr
- Doped AₓF_{y}, with x>0, y>0 and with A = Fe, Mg, Ca, Ta, Cd, Sr, Ba dopant: rare earth
   such as BaF₂:Nd³⁺
- LnF₃ with Ln = rare earth
- LnSF with Ln = rare earth

### 2. Ternary fluorides

- AₓB_{y}F_{z}, with x>0, y>0, z>0 and
   with A = Ba, Sr, K, Pb, Cs, Rb, Li, Na
   with B = Mn, Al, Fe, Cr, Zr, Ga, Tb, Mg
   such as BaMnF₄, SrAlF₅, K₃Fe₅F₁₅, Pb₅Cr₃F₁₉, Ba₂ZrF₈, Pb₂ZrF₈, BaGaF₅, Sr₅Zr₃F₂₂, Ba₃AlF₉, CsAlF₄, BaZr₂F₁₀, K₂FeF₅, Rb₂Cr₅F₁₇, Li₂TbF₆
- CaₓLn_{y}F_{z} with x>0, y>0, z>0 with Ln = rare earth
- Doped AₓB_{y}F_{z}, with x>0, y>0, z>0 and
   with A = Ba, Sr, K, Pb, Cs, Rb, Li, Na
   with B = Mn, Al, Fe, Cr, Zr, Ga, Tb, Mg
   dopant: rare earth
   such as NaMgF₃:Ce³⁺
- (Ln, Ln')SF with Ln, Ln'= rare earth

### 3. Quaternary fluorides

- AₓB_{y}C_{w}F_{z}, with x>0, y>0, w>0, z>0 and
   with A or B = Na, K, Rb, Ca
   with C = Al
   such as NaCaAlF₆, K₂NaAl₃F₁₂, Rb₂NaAl₃F₁₂
- K₂NaAl₃ₙF₉ₙ₊₃, Rb₂NaAl₃ₙF₉ₙ₊₃ with n>0, such as Rb₂NaAl₆F₂₁, RbNiCrF₆, Pb₂MnFeF₉, PbMnFeF₇
- Ln₂AS₂F₄ with A = Ca, Sr and Ln = rare earth, such as Ce₂SrS₂F₄, Sm₂CaS₂F₄
- BaCuSF
- (Y_{0,8}YbₙErₘ)F₃ with n = 0.2 - m and 0.01 ≤ m ≤ 0.02 or n, m are integers

### Oxyfluorides:

### 1. Oxyfluorides based on one metal

- AₓO_{y}F_{z}, with x>0, y>0, z>0 and with A = Nb, Cd such as NbO₂F Cd₄OF₆
- LnOₓF_{y} with Ln = rare earth or Y, with x>0, y>0
- LnOₓS_{y}F_{z} with Ln = rare earth or Y, with x>0, y>0, z>0
- Ln₂Ln'₂O₃F₆, with Ln and Ln'= rare earth or Y

### 2. Oxyfluorides based on two metals

- AₓB_{y}O_{w}F_{z}, with x>0, y>0, w>0, z>0
   with A = K, Na, Pb, Bi, Ba, Li, Cs, Rb
   with B = Mo, W, Ti, B, In, Zr, V, Ta, Nb, Fe, Ni
   such as K₃MoO₃F_{3,} Na₅W₃O₉F₅, Pb₅W₃O₉F₁₀, Bi₂TiO₄F₂, K₃B₃O₃F₆, Na₃B₃O₃F₆, Ba₂lnO₃F, Ba₃ln₂O₅F₂, Ba₂lnO₃F, Li₃TiO₃F, K₃TiOF₅, K₃Zr₂O₂F₇, K₂Zr₂O₂F₆, Cs₂Zr₂O₂F₆, K₂VO₃F K₂V₂O₅F₂, NaMoO₃F, Ba₃V₂O₄F₈ Na₂VOF₅, Li₃NbO₄F, K₂NbOF₅, K₂TaOF₅, Na₃NbO₂F₄ K₃NbO₂F₄, K₃TaO₂F₄, M₂NbOF₅ (M=Li, Na, K, Rb, Cs), Ba₄Nb₂O₃F₁₂, Ba₃Ta₂O₂F₁₂, Ba₄Nb₂O₃F₁₂, Pb₃M₄O₁₂F₂ (M=Nb, Ta), NaNb₃O₅F, NaMoO₃F, Pb₁₋ₓMOₓO₃ₓF₂₋₂ₓ with x<0.15, Pb₃MoO₃F₆, PbMoO₃F₂, Ba₂WO₃F₄, Li₂FeO₂F, LiNiOF, Cs₂WO₂F₄

### 3. Oxyfluorides based on three metals

- AₓB_{y}C_{z}OₙFₘ, with x>0, y>0, z>0, n>0, m>0
   with A = K, Na, Pb, Bi, Ba, Li, Cs, Rb
   with B or C = Mo, W, Ti, B, In, Zr, V, Ta, Nb, Fe, Ni, Th, Sr
   such as ASrNb₂O₆F with A = Li, Na, Rb, WTh₈Zr₁₈O₅₃F₄

Alternatively, the fluorides, but in particular the oxyfluorides and oxysulfofluorides, can also be synthesized by precipitation from metal salts and/or metal alkoxides and fluorides, HF or HF complexes in liquid suspensions, preferably sodium or ammonium fluorides in aqueous systems. For a complete precipitation, a hydrothermal treatment or precipitation may be performed. In contrast to the gas-phase reactions, insolubility of the substances is required to obtain pearlescent pigments according to the present invention.

The new pearlescent pigments can be used as substrate to precipitate further optical layers. If desired, the pigments according to the present invention can be further coated with one or more layers of metal oxides, metal oxide hydrates, metal fluorides and/or semitransparent metal layers on top of the selectively light absorbing layer. The selectively light absorbing layer can also be placed as an intermediate layer of metal oxide, metal oxide hydrate, metal fluoride and/or semitransparent metal stacks. The metal oxide or metal oxide hydrate can be selected from any metal oxide or metal oxide hydrate, preferably from titanium oxide, iron oxide, aluminium oxide, aluminium oxide hydrate, silicon oxide, silicon oxide hydrate, zirconium oxide, chromium oxide, zinc oxide, tin oxide, antimony oxide, indium oxide, potassium ferric ferro cyanides, most preferably from titanium oxide, iron oxide, aluminium oxide, aluminium oxide hydrate, silicon oxide, silicon oxide hydrate and/or mixtures thereof. The metal fluoride is preferably magnesium fluoride. The metal of the semitransparent metal layer can be selected from chromium, molybdenum, aluminium, silver, platinum, nickel, copper and/or gold, preferably from aluminium, silver. In particular, the metal oxide, metal oxide hydrate, metal fluoride and/or semitransparent metal layers are arranged as alternating layers of metal oxide, metal oxide hydrate, metal and/or metal fluoride with a refractive index n > 1.8 and a metal oxide, metal oxide hydrate, metal and/or metal fluoride with a refractive index n ≤ 1.8. Pigments according to this embodiment combine the colour of the selectively light absorbing layer with an intensively lustrous appearance and may show an angle-dependent interference colour.

Preferred examples for metal oxides, metal oxide hydrates and/or metals with a refractive index n > 1.8 are titanium oxide, iron oxide, iron titanate, iron, chromium, silver and/or nickel, preferably titanium oxide, iron oxide, iron titanate.

Preferred examples for metal oxides, metal oxide hydrates, metals and/or metal fluorides with a refractive index n ≤ 1.8 are silicon oxide, silicon oxide hydrate, aluminium oxide, aluminium oxide hydrate, aluminium and/or magnesium fluoride.

Furthermore, the resulting pigments can be coated with inorganic and/or organic compounds to increase their weather stability respectively their photostability. Useful methods are for instance described in US-A 4,134,776, EP-A 0 649 886, WO 97/29059 and references cited therein.

The advantage of this invention is the combination of a great variety of mass-tones of the fluorides and derived compounds with an angle dependent interference color that is adjusted by the layer thickness of the fluoride containing layer. The applications of these new pigments are numerous, such as paints, powder coatings, paper coatings, plastics, cosmetics, inks and security-enhancing features as well as in decorative applications for foods and drugs due to the use of mainly nontoxic materials.

To create new color effects in all applications, the pearlescent pigments according to the present invention can be employed in admixture with filler pigments or transparent and hiding white, colored and black organic and inorganic pigments and also with conventional transparent, colored and black luster pigments based on metal oxide coated mica, TiO₂ flakes, SiO₂ flakes or Al₂O₃ flakes and coated or uncoated metal pigments, BiOCl pigments, platelet-shaped iron oxides or graphite flakes. The inventive pigments can be further coated with organic or inorganic layers to yield combination pigments.

Some fluoride-containing layers of the pigments described in this invention have fluorescent, photoluminescent or electroluminescent properties itself, e.g. Cs₂WO₂F₄ layers. If such a time-delayed color effect is desired, for example in the field of security, optical, projection screen, safety or similar applications, and the inherent property of the invented pigments is not strong enough, physical mixtures of the invented pigments with conventional inorganic or organic fluorescent respectively luminescent pigments can be used.

The pigments and their production process according to the present invention is more illustratively demonstrated but not limited by means of the following examples.

### Examples:

### Example 1:

100 g of muscovite mica flakes (Merck KGaA, diameter 10-50 µm) are suspended in 2 liters of fully deionised water. The suspension is heated to 75°C. 467 ml of a FeCl₃ solution is diluted to 1000 ml with water and is slowly added to the reactor. The pH of the solution is kept at pH 3.1 by addition of 15 % aqueous sodium hydroxide solution. The preparation is filtered off, washed with completely deionised water, dried at 110°C for 12 hours and calcined at 800°C for 30 minutes. As a result, 70 g of Fe₂O₃ are precipitated onto 100 g of mica. This pigment is then put into a fluidized bed reactor, calcined under HF at 700°C during 12 hours. A red FeOF/mica pigment is obtained.

### Example 2:

100 g Iriodin® 504 (Fe₂O₃/mica Merck KGaA, diameter 10-50 µm) are suspended in 2 liters of fully deionised water. The suspension is heated to 75°C. A CeCl₃ solution (126 g diluted in 600 ml water) is slowly added to the reactor. The solution is kept at pH 7.5 by simultaneous addition of 20 % aqueous sodium hydroxide solution. The preparation is filtered off, washed with completely deionised water and dried at 110°C for 12 hours. As a result, 50 g of CeO₂ are precipitated onto 100 g of Iriodin® 504. The pigment is then put into a fluidized bed reactor. The precursor is fluidized with N₂ to a temperature in the range of from 750 to 850°C and then is treated with HF for 360 minutes. A pigment with a reddish golden color (mixed iron oxyfluoride) and a yellow shade (cerium oxyfluoride) is obtained.

### Example 3:

100 g of muscovite mica flakes (Merck KGaA, diameter 10-50 µm) are suspended in 2 liters of fully deionised water. The suspension is heated to 75°C. A CeCl₃ solution (126 g diluted in 600 ml water) is slowly added into the reactor. The solution is kept at pH 7.5 by addition of 20 % aqueous sodium hydroxide solution. The preparation is filtered off, washed with completely deionised water, dried at 110°C for 12 hours. As a result, 50 g of hydrous CeO₂ are precipitated onto 100 g of muscovite mica flakes. The pigment is then put into a fluidized bed reactor. The precursor is fluidized with N₂ to temperatures of from 750 to 850°C and then is converted with HF for 360 minutes. A pigment with a yellow color (cerium oxyfluoride) is obtained.

### Example 4:

50 g of CeO₂:VCl₃(10:1wt.-ratio) are precipitated onto 100 g of muscovite mica flakes (Merck KGaA, diameter 10-50 µm) using a CeCl₃ solution (126 g of CeCl₃ solution containing 12.6 g of VCl₃ diluted into 600 ml of water) as described in example 3. The dried pigment is then put into the fluidized bed, calcined at 800°C under HF for 360 minutes. A pigment with a blue color (vanadium-doped cerium oxyfluoride) was obtained.

### Example 5:

A metallized cerium oxyfluoride pigment is produced by thermally decomposing chromium hexacarbonyl in the presence of heated cerium oxyfluoride coated onto muscovite mica flakes as described in the example 3. This pigment is fluidized with nitrogen to achieve and maintain a non-bubbling fluidized bed and an oxygen free atmosphere. Then the reactor is heated to 400 to 450°C and kept under this condition throughout the following coating process. A stream of nitrogen loaded with chromium hexacarbonyl is prepared by passing nitrogen through a flask containing chromium hexacarbonyl, which is kept at 80°C, and introduced subsequently into the reactor. The vaporized compound is passed into the tube for about 90 minutes. About 5 nm of chromium is deposited on the cerium oxyfluoride pigment based onto muscovite mica flakes, forming a semitransparent layer. The organic by-product of the decomposition reaction is separated from the pigment into a scrubber.

### Example 6:

100 g of muscovite mica flakes (Merck KGaA, diameter 10-50 µm) are suspended in 2 liters of fully deionised water. The suspension is heated to 75°C. A CeCl₃ solution (126 g diluted in 600 ml water) is slowly added into the reactor. The solution is kept at pH 7.5 by addition of 20 % aqueous sodium hydroxide solution. The preparation is filtered off, washed with completely deionised water, dried at 110°C for 12 hours. As the result, 50 g of hydrous CeO₂ are precipitated onto 100 g muscovite mica. The pigment is then put into a fluidized bed reactor. The precursor is fluidized with nitrogen at 850°C for 30 minutes and then converted by HF for 360 minutes. This pigment is then put into a fluidized bed reactor, calcined under H₂S at 700°C during 2 hours. The resulting intermediate is put into a fluidized bed reactor, calcined under HF at 700°C during 2 hours. A red cerium fluorosulfide onto muscovite mica pigment is obtained.

## Claims

1. Pearlescent pigments based on substrates comprising at least one selectively light absorbing layer which consists of a fluoride, oxyfluoride, fluorosulfide and/or oxyfluorosulfide.

2. Pearlescent pigments according to claim 1, wherein the substrate is platelet-shaped, spherical or needle-shaped.

3. Pearlescent pigments according to claim 2, wherein the substrate is platelet-shaped and is of mica, SiO₂, aluminum oxide, glass, micaceous iron oxide, oxidized graphite, aluminum oxide-coated graphite, pearlescent pigments or pearlescent multilayer pigments, or of coated or uncoated SiO₂-spheres or needle-shaped iron oxides.

4. Pearlescent pigments according to any of claims 1 to 3, **characterized in that** the selectively light absorbing layer is a binary fluoride or fluorosulfide, a ternary fluoride or fluorosulfide, a quaternary fluoride or fluorosulfide or an oxyfluoride or oxyfluorosulfide based on one, two or three metals.

5. Pearlescent pigments according to any of claims 1 to 4, **characterized in that** the thickness of the selectively light absorbing layer is between 5 and 500 nm.

6. Pearlescent pigments according to any of claims 1 to 5, **characterized in that** the pigments are further coated on top of the selectively light absorbing layer with one or more layers of metal oxides, metal oxide hydrates, metal fluorides and/or semitransparent metal layers.

7. Pearlescent pigments according to claim 6, **characterized in that** the metal oxide is selected from TiO₂, iron oxide and/or mixtures thereof and the metal is selected from Al, Mo and/or Cr.

8. Process for the preparation of a pigment according to claim 1 comprising precipitating a layer of an oxide, hydroxide, mixed oxide and/or mixed hydroxide onto a substrate and then converting the oxide, hydroxide, mixed oxide and/or mixed hydroxide into a fluoride, oxyfluoride, fluorosulfide and/or, oxyfluorosulfide.

9. Process according to claim 8, **characterized in that** the conversion is carried out in a fluidized bed reactor.

10. Process according to claim 8 or 9, **characterized in that** the conversion is carried out with a reactive gas which consists of fluorine and/or HF, alone or in combination with H₂S, CS₂, sulfur or a mixture thereof.

11. Process according to claim 10, **characterized in that** additionally an inert gas such as Ar or N₂ is present during the conversion.

12. Process according to claim 8, **characterized in that** the substrate is platelet-shaped and is of mica, SiO₂, aluminum oxide, glass, micaceous iron oxide, oxidized graphite, aluminum oxide-coated graphite, pearlescent pigments or pearlescent multilayer pigments, or of coated or uncoated SiO₂-spheres or needle-shaped iron oxides.

13. Process according to claim 8, **characterized in that** the oxide, hydroxide, mixed oxide and/or mixed hydroxide is doped with metal ions, silicon oxide, aluminum oxide, sulfur, phosphate ions and/or sulfate ions.

14. Process according to claim 13, **characterized in that** the metals are selected from silicon, vanadium, chromium, aluminum, cerium, neodymium, praseodymium, selenium, cobalt, nickel and/or zinc.

15. Use of a pigment according to claim 1 in paints, powder coatings, paper coatings, plastics, cosmetics, inks, decorative applications for foods and drugs and security-enhancing features.

16. Use of a pigment according to claim 1 as phosphorescent, fluorescent or luminescent materials in security, optical or projection screen applications.
